# EUROPEAN PATENT APPLICATION

(11) **EP 1 364 654 A1**
(43) Date of publication of application: **26.11.2003**
(21) Application number: 02700680.8
(22) Date of filing: 21.02.2002
(51) Int. Cl.: A61K 38/17, A61K 38/36, A61K 9/08, A61K 47/10, A61P 27/02

(54) **EYE DROPS**

(30) Priority: 26.02.2001 JP 2001050297
(71) Applicant: Kowa Company, Ltd., Nagoya-shi, Aichi 460-0003 (JP); Juridical Foundation, The Chemo-Sero-Therapeutic Research Institute, Kumamoto-shi, Kumamoto 860-8568 (JP)
(72) Inventor: NARUSE, Haruhiko, Fujinomiya-shi, Shizuoka 418-0022 (JP); SANO, Misato, Fujieda-shi, Shizuoka 426-0005 (JP); SHINODA, Yasuo, Shizuoka-shi, Shizuoka 420-0867 (JP); INAGI, Toshio, Mishima-shi, Shizuoka 411-0038 (JP)
(74) Representative: Bannerman, David Gardner
(86) International application number: JP0201563
(87) International publication number: WO02067977

(57) **Abstract**

An object of present invention is to provide an eye drop which causes no uncomfortable irritation upon instillation and shows favorable long-term storage stability of CPB-I. The eye drop which comprises CPB-I and a polyhydric alcohol, preferably the polyhydric alcohol having a carbonyl value of 5 µmol/g or lower, can be provided.

## Description

### Technical Field

The present invention relates to an eye drop which causes no uncomfortable irritation upon instillation and shows favorable long-term CPB-I storage stability, and a method for producing the same.

### Background Art

CPB-I (a.k.a. "annexin V") is a substance that is extensively distributed in tissues and secretions of living bodies including human placenta (Chem. Pharm. Bull, 38, 1957-1960, 1990), present in cytoplasm in a cell and has physiological activities such as an anticoagulation action. It has been reported that CPB-I can be extracted from organs of human or other animals (Japanese Patent Laid-open Publication (Kokai) No. 62-174023), and expressed by gene recombination techniques (Japanese Patent Laid-open Publication (Kokai) No. 64-20095). It has also been reported to use CPB-I as a therapeutic agent for corneal diseases (WO92/08475).

Meanwhile, salts such as sodium chloride or potassium chloride are generally mixed in eye drops as an isotonic agent to eliminate uncomfortable stimuli to eyes upon instillation. However, if the aforementioned salts are mixed in an eye drop containing CPB-I, long-term CPB-I storage stability undesirably becomes problematic. In order to stabilize CPB-I, it is contemplated to mix ethylenediaminetetraacetic acid (EDTA), which is an antioxidant, or a salt thereof, or ascorbic acid. However, EDTA or a salt thereof may inhibit the wound healing action of CPB-I, and ascorbic acid is not preferred either since the pH range in which it is stable is different from that of CPB-I.

So far, there have known methods of stabilizing CPB-I by mixing a basic amino acid or saccharide upon isolation, purification, lyophilization or the like (Japanese Patent Laid-open Publication (Kokai) Nos. 4-198195 and 4-198196). However, long-term storage stability of eye drops has not been examined. That is, it is difficult to prepare an eye drop that causes no uncomfortable irritation upon instillation and has favorable long-term CPB-I storage stability, and hence no satisfactory products have been obtained so far.

### Disclosure of the Invention

The present invention was accomplished from the aforementioned viewpoint, and its object is to provide an eye drop containing CPB-I, which causes no uncomfortable irritation upon instillation and shows favorable long-term storage stability of CPB-I, and a method for producing the same.

The inventors of the present invention assiduously studied in consideration of the above, and as a result, they found a composition of eye drop that causes no uncomfortable irritation upon instillation and shows favorable long-term storage stability of CPB-I. That is, they found that an eye drop containing CPB-I causing no uncomfortable irritation upon instillation and showing favorable long-term storage stability of CPB-I could be provided by adding a polyhydric alcohol as an isotonic agent to the eye drop, and thus accomplished the present invention. Further, they found that an eye drop having further excellent long-term storage stability of CPB-I could be provided by using a polyhydric alcohol having a low carbonyl value as the polyhydric alcohol, and accomplished the present invention.

That is, the present invention provides the followings.
(1) An eye drop containing CPB-I and a polyhydric alcohol.
(2) The eye drop according to (1), wherein the polyhydric alcohol is selected from glycerin, propylene glycol, mannitol and sorbitol.
(3) The eye drop according to (1) or (2), wherein the polyhydric alcohol has a carbonyl value of 5 µmol/g or lower.
(4) A method for producing an eye drop containing CPB-I, which comprises mixing a polyhydric alcohol having a carbonyl value of 5 µmol/g or lower in the eye drop together with CPB-I.

Hereafter, the present invention will be described in detail.

The eye drop of the present invention contains CPB-I as an active ingredient. As CPB-I, human-derived CPB-I can be mentioned. The amino acid sequence of human CPB-I has already been reported (Japanese Patent Laid-Open Publication (Kokai) No. 64-20095). Human CPB-I has the amino acid sequence of SEQ ID NO: 1 mentioned in

### Sequence Listing.

Further, CPB-I typically has the following properties.

### <Properties>

(i) Molecular weight (determined by SDS-polyacrylamide gel electrophoresis in a reduced state): 34,000 ± 2,000
(ii) Isoelectric point (determined by isoelectric point column electrophoresis using ampholyte): 4.7 ± 0.1
(iii) Stability
   (a) Inactivated by heat treatment at 50°C for 30 minutes
   (b) Stable at pH 4 to 10
   (c) Stable in plasma at 37°C for 30 minutes
(iv) Action on blood coagulation system
   (a) Extends coagulation time after further addition of calcium
   (b) Extends prothrombin time
   (c) Extends activated partial thromboplastin time
(v) Presence of aspartic acid, threonine, serine, glutamic acid, proline, glycine, alanine, 1/2 cystine, valine, methionine, isoleucine, leucine, tyrosine, phenylalanine, histidine, lysine and arginine is observed in amino acid analysis.

The origin, presence or absence of sugar chain and the like of CPB-I used in the present invention are not particularly limited so long as it shows an effect as an active ingredient of an eye drop such as therapeutic effect on cornea diseases. Further, derivatives thereof subjected to alteration or modification such as amino acid substitution may also be used so long as they show the aforementioned effect.

Although CPB-I used in the present invention is preferably human CPB-I, it may be a protein comprising an amino acid sequence of SEQ ID NO: 1 including substitution, deletion, insertion or addition of one or more amino acid residues or an amino acid sequence having a substituent at its C- or N-terminus so long as it shows the aforementioned effect as an active ingredient.

CPB-I can be extracted and purified from a human or animal organ containing CPB-I (Japanese Patent Laid-Open Publication (Kokai) No. 62-174023). However, it is preferably produced by a recombinant DNA technique using DNA coding for CPB-I in view of mass production. DNA coding for the CPB-I protein has already been cloned, and its nucleotide sequence has been elucidated (Japanese Patent Laid-open Publication (Kokai) No. 64-20095). Further, a method for producing CPB-I using this DNA has been described, and CPB-I produced by this method can be preferably used in the present invention.

DNA coding for CPB-I of the present invention is not particularly limited so long as the encoded amino acid sequence satisfies the aforementioned requirements. Specific examples thereof include DNAs having the nucleotide sequence described in Japanese Patent Laid-open Publication (Kokai) No. 64-20095.

Since the amino acid sequence of the CPB-I protein and the nucleotide sequence of DNA coding for it have already been elucidated, DNA coding for CPB-I protein described above can be obtained from a human or animal chromosomal DNA or chromosome library by PCR using primers prepared based on the sequence.

Methods for preparation of DNA coding for CPB-I, and preparation of chromosomal DNA, preparation of a chromosomal DNA library, hybridization, PCR, preparation of plasmid DNA, digestion and ligation of DNA, transformation and so forth in the preparation of CPB-I using recombinant DNA techniques are described in Japanese Patent Laid-Open Publication (Kokai) No. 64-20095.

The content of CPB-I used in the present invention is 0.0001 to 1% by weight, preferably 0.001 to 0.1% by weight, particularly preferably 0.003 to 0.03% by weight, based on the total weight of the eye drop.

The eye drop of the present invention contains a polyhydric alcohol together with CPB-I. As the polyhydric alcohol, dihydric to hexahydric alcohols are preferred. Specific examples thereof include glycerin, propylene glycol, mannitol, sorbitol and so forth, and glycerin is preferred. The content of polyhydric alcohol is preferably about such an amount that the eye drop composition should become isotonic. Specifically, the content is 0.1 to 20% by weight, preferably 1 to 10% by weight, particularly preferably 1.5 to 5% by weight, based on the total weight of the eye drop.

If content of an oxidized alcohol contained in the polyhydric alcohol, specifically a carbonyl compound, is high, the CPB-I activity may decline during the storage of the eye drop. Therefore, a polyhydric alcohol having a low carbonyl value is preferably used. Specifically, the carbonyl value is preferably a value lower than the value shown in the examples later, for example, 5 µmol/g or lower, more preferably 3 µmol/g or lower, further preferably 1 µmol/g or lower.

The carbonyl value of polyhydric alcohol used in the present invention referred to herein means a number of µmol of the carbonyl compound in 1 g of a sample, which is obtained by, for example, a spectrometric method performed after color development with 2,4-dinitrophenylhydrazine under a certain condition (A.S. Heick, M.F. Benca, Jr. Mitchell, JAOCS, 31, 88, 1954; S. Mitsunaga & U. Shimamura, Oil Chemistry, 7, 275, 1958). Hereafter, a general method for measuring the carbonyl value will be described.

### <Method for measuring carbonyl value>

In an amount of 1 g of concentrated glycerin is weighed and added with ethanol (aldehyde free) to a total volume of 50 ml. In an amount of 5 ml of the prepared solution is collected in a 25-ml measuring flask, added with 2 ml of 3.6% (w/v) solution of trichloroacetic acid (dissolved in toluene) and 3 ml of 0.05% (w/v) solution of 2,4-dinitrophenylhydrazine (dissolved in toluene), stirred, allowed to react in a thermostat at 60 ± 1°C for 30 minutes, and then left until cooled to room temperature. The mixture is further added with 5 ml of 4% (w/v) solution of potassium hydroxide in ethanol (aldehyde free) and then toluene to a total volume of 25 ml. Following 10 minutes after the addition of the potassium hydroxide solution, the absorbance is measured at 435 nm. A blank test is performed without adding concentrated glycerin for correction. In amounts of 1, 3, 5 and 7 ml of aldehyde standard stock solution (obtained by dissolving 0.142 g of nonanal [Wako Pure Chemical Industries] in toluene in a total volume of 100 ml and then adding toluene to 5 ml of this solution to a total volume of 100 ml) is collected and each added with aldehyde-free ethanol to a total volume of 25 ml. In a volume of 5 ml of each solution is collected in a 25-ml measuring flask, and the same operations as in the aforementioned standard test method were performed for the solution to create a calibration curve.

The eye drop of the present invention can contain additives generally used, for example, buffers, thickeners, stabilizers, nonionic surfactants and so forth.

Examples of the buffers include boric acid, borax, citric acid, primary phosphate, acetate, secondary phosphate, hydrogencarbonate, carbonate and so forth. The content of the buffer is 0.01 to 2% by weight, preferably 0.05 to 1% by weight, particularly preferably 0.1 to 0.5% by weight, based on the total weight of the eye drop. The eye drop preferably has pH of 5 to 10, more preferably 6 to 9.

Examples of the thickeners include polyvinyl alcohol, polyvinylpyrrolidone, carboxyvinyl polymer and so forth. The thickeners prevent the eye drop from running out from the eye and extend the retention time in the eye to provide higher drug efficacy. Further, if the CPB-I eye drop is shaken, CPB-I may precipitate. However, precipitation of CPB-I is prevented by addition of a thickener. The content of the thickener is 0.01 to 2% by weight, preferably 0.025 to 1% by weight, particularly preferably 0.05 to 0.5% by weight, based on the total weight of the eye drop.

Examples of the stabilizers include sodium caprylate, arginine, sodium sulfite, sodium hydrogensulfite and so forth. The content of the stabilizer is 0.01 to 2% by weight, preferably 0.05 to 1% by weight, particularly preferably 0.1 to 0.5% by weight, based on the total weight of the eye drop.

Examples of the nonionic surfactants include fatty acid polyhydric alcohol esters, polyoxyethylene sorbitan fatty acid esters, polyoxyethylene ethers such as polyoxyethylene hydrogenated caster oil, ethylene oxide adducts such as alkylpolyoxyethylene ethers, alkylcarbonylpolyoxyethylenes and N,N'-di(polyoxyethylene)alkane amides, fatty acid sucrose esters, N,N'-di(alkanol)alkane amides, polyoxyethylene block copolymers and so forth. The content of the nonionic surfactant is 0.01 to 5% by weight, preferably 0.02 to 2% by weight, particularly preferably 0.03 to 1% by weight, based on the total weight of the eye drop.

In view of long-term storage stability of CPB-I, it is preferable not to use salts generally used as an isotonic agent of eye drops such as sodium chloride or potassium chloride, or the content thereof is preferably reduced to such an extent that stability should not be degraded.

The eye drop of the present invention is an eye drop solution preparation. This preparation can be prepared as a preparation to be dissolved or diluted upon use. When the eye drop is prepared as a preparation to be dissolved upon use, CPB-I and polyhydric alcohol may be separately packaged.

### Brief Description of the Drawings

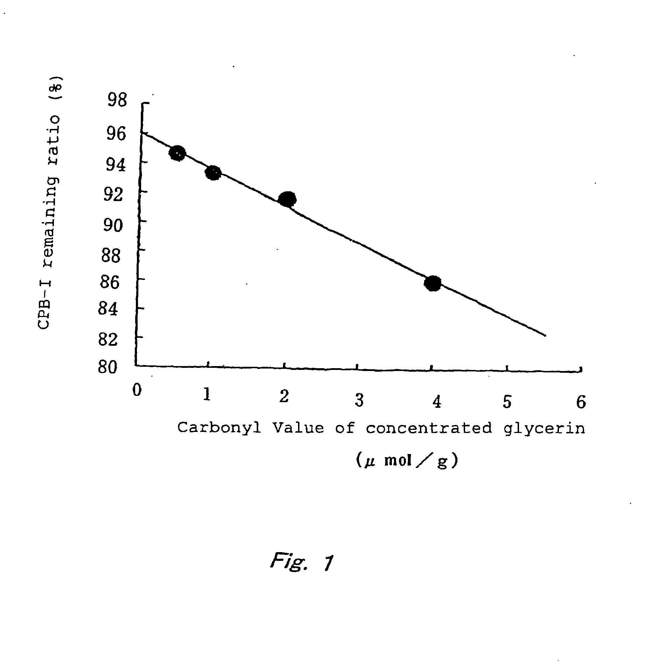

Fig. 1 shows influence of the carbonyl value of polyhydric alcohol on CPB-I stability.

### Best Mode for Carrying out the Invention

The present invention will be explained more specifically with reference to the following examples.

The eye drops described in the examples and comparative examples described below were prepared by appropriately adding boric acid and borax or disodium monohydrogenphosphate and monosodium dihydrogenphosphate dihydrate to adjust all the preparations to pH 7.5. Concentrated glycerin, propylene glycol, sorbitol, sodium chloride and potassium chloride were appropriately added to adjust osmotic pressures of all the preparations to 286 m Osm.

### Example 1

In an amount of 0.01 g of CPB-I, 2.3 g of concentrated glycerin, 0.11 g of boric acid, 0.085 g of borax, 0.05 g of polyoxyethylene sorbitan fatty acid ester and 0.1 g of polyvinylpyrrolidone were added with sterile purified water to a total volume of 100 mL and dissolved by stirring.

### Example 2

Preparation was performed in the same manner as in Example 1 except that 2.1 g of propylene glycol was added instead of 2.3 g of concentrated glycerin, and 0.016 g of borax was added instead of 0.085 g of borax.

### Example 3

Preparation was performed in the same manner as in Example 1 except that 4.0 g of sorbitol was added instead of 2.3 g of concentrated glycerin, and 0.26 g of disodium monohydrogenphosphate and 0.05 g of monosodium dihydrogenphosphate dihydrate were added instead of boric acid and borax.

### Examples 4 to 6

Preparation was performed in the same manner as in Example 1 except that concentrated glycerin having a different carbonyl value was used.

### Comparative Example 1

Preparation was performed in the same manner as in Example 1 except that 0.8 g of sodium chloride was added instead of 2.3 g of concentrated glycerin, and 0.011 g of borax was added instead of 0.085 g of borax.

### Comparative Example 2

Preparation was performed in the same manner as in Example 1 except that 1.0 g of potassium chloride was added instead of 2.3 g of concentrated glycerin, and 0.011 g of borax was added instead of 0.085 g of borax.

### <Evaluation of eye drops of examples and comparative examples>

Stability of CPB-I was examined in the eye drops of Examples 1 to 6 and Comparative Examples 1 and 2. Stability of CPB-I was measured as remaining ratio of CPB-I (based on the ratio immediately after production, which was taken as 100%) after the preparations were stored at 45°C for 2 weeks.

The results are shown in Tables 1, 2 and Fig. 1. Fig. 1 shows a graph of the results shown in Table 2.

**Table 1**

| | Example 1 | Example 2 | Example 3 | Comparative Example 1 | Comparative Example 2 |
|---|---|---|---|---|---|
| CPB-I | 0.01 | 0.01 | 0.01 | 0.01 | 0.01 |
| Concentrated glycerin | 2.3 | - | - | - | - |
| Propylene glycol | - | 2.1 | - | - | - |
| Sorbitol | - | - | 4.0 | - | - |
| Sodium chloride | - | - | - | 0.8 | - |
| Potassium chloride | - | - | - | - | 1.0 |
| Disodium monohydrogen-phosphate | - | - | 0.26 | - | - |
| Monosodium dihydrogen-phosphate dihydrate | - | - | 0.05 | - | - |
| Boric acid | 0.11 | 0.11 | - | 0.11 | 0.11 |
| Borax | 0.085 | 0.016 | - | 0.011 | 0.011 |
| Polyoxyethylene sorbitan fatty acid ester | 0.05 | 0.05 | 0.05 | 0.05 | 0.05 |
| Polyvinyl-pyrrolidone | 0.1 | 0.1 | 0.1 | 0.1 | 0.1 |
| Sterile purified water | Appropriate amount to make total 100 mL | Appropriate amount to make total 100 mL | Appropriate amount to make total 100 mL | Appropriate amount to make total 100 mL | Appropriate amount to make total 100 mL |
| CPB-I remaining ratio (%) after storage at 45°C for 2 weeks | 95 | 95 | 93 | 44 | 39 |

**Table 2**

| | Example 1 | Example 4 | Example 5 | Example 6 |
|---|---|---|---|---|
| Carbonyl value of concentrated glycerin (µmol/g) | 0.5 | 1 | 2 | 4 |
| CPB-I remaining ratio (%) after storage at 45°C for 2 weeks | 95 | 93 | 92 | 86 |

As described above, it was confirmed from the results of Examples 1 to 3 and Comparative Examples 1 and 2 that eye drops having favorable long-term CPB-I stability could be obtained if a polyhydric alcohol was added to the eye drops as an isotonic agent.

Further, it was confirmed from the results of Examples 1 and 4 to 6 that as the carbonyl value of polyhydric alcohol became lower, the long-term CPB-I stability in the eye drops became better.

### Industrial Applicability

According to the present invention, an eye drop which causes no uncomfortable irritation upon instillation and has favorable long-term storage stability of CPB-I can be provided.

## Claims

1. An eye drop containing CPB-I and a polyhydric alcohol.

2. The eye drop according to claim 1, wherein the polyhydric alcohol is selected from the group consisting of glycerin, propylene glycol, mannitol and sorbitol.

3. The eye drop according to claim 1 or 2, wherein the polyhydric alcohol has a carbonyl value of 5 µmol/g or lower.

4. A method for producing an eye drop containing CPB-I, which comprises mixing a polyhydric alcohol having a carbonyl value of 5 µmol/g or lower in the eye drop together with CPB-I.


SEQUENCE LISTING

<110> KOWA CO., LTD.
      JURIDICAL FOUNDATION THE CHEMO-SERO-THERAPEUTIC RESEARCH INSTITUTE

<120> Eye Drop

<130> K-650P1311

<150> JP 2001-50297
<151> 2001-02-26

<160> 1

<170> PatentIn Ver. 2.0

<210> 1
<211> 319
<212> PRT
<213> Homo sapiens

<400> 1
Ala Gln Val Leu Arg Gly Thr Val Thr Asp Phe Pro Gly Phe Asp Glu
  1               5                  10                  15
Arg Ala Asp Ala Glu Thr Leu Arg Lys Ala Met Lys Gly Leu Gly Thr
             20                  25                  30
Asp Glu Glu Ser Ile Leu Thr Leu Leu Thr Ser Arg Ser Asn Ala Gln
         35                  40                  45
Arg Gln Glu Ile Ser Ala Ala Phe Lys Thr Leu Phe Gly Arg Asp Leu
     50                  55                  60
Leu Asp Asp Leu Lys Ser Glu Leu Thr Gly Lys Phe Glu Lys Leu Ile
 65                  70                  75                  80
Val Ala Leu Met Lys Pro Ser Arg Leu Tyr Asp Ala Tyr Glu Leu Lys
                 85                  90                  95
His Ala Leu Lys Gly Ala Gly Thr Asn Glu Lys Val Leu Thr Glu Ile
            100                 105                 110
Ile Ala Ser Arg Thr Pro Glu Glu Leu Arg Ala Ile Lys Gln Val Tyr
        115                 120                 125
Glu Glu Glu Tyr Gly Ser Ser Leu Glu Asp Asp Val Val Gly Asp Thr
    130                 135                 140
Ser Gly Tyr Tyr Gln Arg Met Leu Val Val Leu Leu Gln Ala Asn Arg
145                 150                 155                 160

Asp Pro Asp Ala Gly Ile Asp Glu Ala Gln Val Glu Gln Asp Ala Gln
                165                 170                 175
Ala Leu Phe Gln Ala Gly Glu Leu Lys Trp Gly Thr Asp Glu Glu Lys
            180                 185                 190
Phe Ile Thr Ile Phe Gly Thr Arg Ser Val Ser His Leu Arg Lys Val
        195                 200                 205
Phe Asp Lys Tyr Met Thr Ile Ser Gly Phe Gln Ile Glu Glu Thr Ile
    210                 215                 220
Asp Arg Glu Thr Ser Gly Asn Leu Glu Gln Leu Leu Leu Ala Val Val
225                 230                 235                 240
Lys Ser Ile Arg Ser Ile Pro Ala Tyr Leu Ala Glu Thr Leu Tyr Tyr
                245                 250                 255
Ala Met Lys Gly Ala Gly Thr Asp Asp His Thr Leu Ile Arg Val Met
            260                 265                 270
Val Ser Arg Ser Glu Ile Asp Leu Phe Asn Ile Arg Lys Glu Phe Arg
        275                 280                 285
Lys Asn Phe Ala Thr Ser Leu Tyr Ser Met Ile Lys Gly Asp Thr Ser
    290                 295                 300
Gly Asp Tyr Lys Lys Ala Leu Leu Leu Leu Cys Gly Glu Asp Asp
305                 310                 315